(19) Europäisches Patentamt
European Patent Office
Office européen des brevets

(11) **EP 3 628 220 A1**

(12) **EUROPEAN PATENT APPLICATION**

(43) Date of publication:
01.04.2020 Bulletin 2020/14

(51) Int Cl.:
*A61B 5/022* (2006.01)     *A61B 5/0225* (2006.01)
*A61B 5/0235* (2006.01)

(21) Application number: 18196774.6

(22) Date of filing: 26.09.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• KUENEN, Maarten Petrus Joseph
  **5656 AE Eindhoven (NL)**
• LAMICHHANE, Bishal
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **A CUFF FOR USE WITH AN INFLATION-BASED NON-INVASIVE BLOOD PRESSURE MEASUREMENT APPARATUS**

(57)     There is provided a cuff (10) for use with an inflation-based non-invasive blood pressure, NIBP, measurement apparatus. The cuff (10) comprises an inlet (12) configured to be coupled to an outlet of the inflation-based NIBP measurement apparatus to receive a flow of fluid and a bladder (14) coupled to the inlet (12) and inflatable to pressurize a measurement site of a subject by receiving the flow of fluid. The cuff (10) also comprises a valve (16) disposed along a flow path between the inlet (12) and the bladder (14) or disposed on the bladder (14). The flow resistance of the valve (16) is such that the valve (16) passes part of the flow of fluid received in the bladder (14) to the atmosphere in order to inflate the bladder (14) at a required flow rate for inflating the cuff.

Fig. 1

**Description**

FIELD OF THE INVENTION

The disclosure relates to a cuff for use with an inflation-based non-invasive blood pressure (NIBP) measurement apparatus.

BACKGROUND OF THE INVENTION

Blood pressure (BP) or, more precisely, arterial blood pressure, is the pressure exerted by circulating blood on the arterial vessel walls. It is one of the key vital signs to establish patient well-being and therefore needs to be monitored for patients at risk. Blood pressure is a periodic signal, which rises at each contraction of the heart and decreases in between heart beats. It is typically described by systolic blood pressure (SBP), diastolic blood pressure (DBP) and mean arterial blood pressure (MAP), where systolic blood pressure is the maximum blood pressure during heart cycle, diastolic blood pressure is the minimum blood pressure during heart cycle, and mean arterial blood pressure is the average blood pressure during a heart cycle.

Different techniques exist by which blood pressure can be determined and these can be classified as invasive or non-invasive blood pressure measurement techniques. Typically, non-invasive blood pressure (NIBP) measurement techniques are cuff-based, which require an inflatable cuff to be placed around a limb (which is usually the upper arm) of a subject. The pressure in the cuff is then changed to infer blood pressure. There are two common methods that use a cuff in this way, which are referred to in the art as the auscultatory method and the oscillometric method respectively.

The auscultatory method for NIBP measurement is based on the appearance and disappearance of sounds created by the artery under the cuff during the period that the cuff pressure is changed. These sounds are referred to in the art as Korotkoff sounds. The pressures at which the Korotkoff sounds appear and vanish are indicative of DBP and SBP with Korotkoff sounds appearing at each heart beat between DBP and SBP. The measurement of sound can be performed manually with a stethoscope that is placed over the artery just below the cuff, or in an automated way with a microphone under the cuff.

In the oscillometric method for NIBP measurement, the systolic and diastolic blood pressure values are based on small volume oscillations or pressure oscillations that are induced in the cuff by each heart beat. The amplitude of these volume or pressure oscillations depends on the difference between the cuff pressure and the actual arterial blood pressure. Systolic blood pressure and diastolic blood pressure are then determined as the cuff pressure where the volume or pressure oscillations have amplitudes of a certain fraction of the maximum oscillation amplitude. These fractions are typically heuristically determined.

In both the auscultatory and oscillometric method, the mean arterial pressure is typically calculated as: MAP = 2/3*DBP + 1/3*SBP.

The oscillometric and auscultatory measurement methods can be performed either during inflation of the cuff or during deflation of the cuff. Conventionally, measurements during deflation are used, in which the cuff is rapidly inflated to a level above the SBP where the blood flow in the artery under the cuff is blocked, after which cuff pressure is decreased gradually or in a stepwise manner. During deflation, the volume or pressure oscillations or the Korotkoff sounds are measured. While deflation stage NIBP measurement is well-established, an issue exists in the discomfort it introduces to the subject. In particular, the subject is exposed to a relatively high cuff pressure for a certain amount of time and pressures above a certain level can be uncomfortable and even painful, either due to the pressure exerted by the cuff itself or due to a build-up of venous blood in the clamped extremity (namely, venous pooling). The longer these pressures are applied to the subject, the higher the discomfort level is for the subject.

Another issue with utilizing the deflation based NIBP measurement is that the process of inflating the cuff and then deflating the cuff can be considerably long, where each NIBP measurement during deflation typically takes 40 seconds to complete. Also, since a defined maximum pressure level needs to be achieved before the deflation procedure can be initiated, the subject is exposed to a maximum cuff pressure that is higher than that required for the blood pressure measurement itself. Furthermore, the inherent variability of blood pressure over time can distort a single blood pressure measurement.

Due to these issues, apparatus have been developed that modify cuff-based NIBP measurements to determine the arterial volume (or pressure) oscillations during (e.g. continuous) inflation of the cuff. An example of such apparatus are inflation based NIBP (iNIBP) apparatus that use a fixed flow (mL/s) and variable speed (mmHg/s) or a fixed speed (mmHg/s) and a variable flow (mL/s) to inflate the cuff. However, the fixed flow technique results in the iNIBP measurement apparatus only functioning for a small range of cuffs as, for smaller cuffs, the inflation is too fast (which results in there being too few pressure oscillations to have an accurate estimate of systolic and diastolic blood pressure) and, for larger cuffs, the iNIBP measurement becomes slow. The fixed speed technique overcomes these problems by changing the flow for a certain desired speed. However, there still exists an issue with this implementation in that a wide range of flows has to be generated and thus a wide range of cuffs have to be available for use with the iNIBP measurement apparatus.

The pump of the iNIBP measurement apparatus can be used to generate a range of flows. However, it is difficult to span a complete flow range from the smallest of cuffs (e.g. cuffs for neonatal subjects) to the largest of cuffs (e.g. thigh cuffs for an adult) with a normal pump. Moreover, currently, the only techniques aimed at addressing this require additional hardware components to be added to the iNIBP measurement apparatus, which can be complex.

## SUMMARY OF THE INVENTION

As noted above, the limitations associated with existing techniques are that the range of flows that can be generated to inflate a cuff for use in inflation-based non-invasive blood pressure (iNIBP) measurement is limited and the limited range of flows that are possible also require complex hardware modifications to an iNIBP measurement apparatus for use with the cuff. It would thus be valuable to address these limitations.

Therefore, according to a first aspect, there is provided a cuff for use with an inflation-based non-invasive blood pressure, NIBP, measurement apparatus. The cuff comprises an inlet configured to be coupled to an outlet of the inflation-based NIBP measurement apparatus to receive a flow of fluid and a bladder coupled to the inlet and inflatable to pressurize a measurement site of a subject by receiving the flow of fluid. The cuff also comprises a valve disposed along a flow path between the inlet and the bladder or disposed on the bladder. The flow resistance of the valve is such that the valve passes part of the flow of fluid received in the bladder to the atmosphere in order to inflate the bladder at a required flow rate for inflating the cuff.

In some embodiments, the flow resistance of the valve may be defined based on a physical property of the cuff. In some embodiments, the physical property of the cuff may comprise any one or more of a size of the cuff, an elasticity of the cuff, and a compliance of the cuff. In some embodiments, the flow resistance of the valve may be defined based on a flow range of a pump configured to output the flow of fluid. In some embodiments, the flow resistance of the valve may be defined based on a target inflation rate for the cuff to reach for determining a blood pressure measurement for the subject. In some embodiments, the valve may have a diameter that defines the flow resistance of the valve. In some embodiments, the valve may be a needle valve, a globe valve, a butterfly valve, or a poppet valve. In some embodiments, the cuff may comprise a deflation valve controllable to deflate the bladder.

According to a second aspect, there is provided a system comprising at least one cuff as described earlier. In some embodiments, the system may comprise the inflation-based NIBP measurement apparatus. In some embodiments, the inflation-based NIBP measurement apparatus may comprise a deflation valve controllable to deflate the bladder. In some embodiments, the inflation-based NIBP measurement apparatus may comprise a pump configured to output the flow of fluid. In some embodiments, the system may comprise a processor configured to acquire a signal indicative of pressure oscillations detected in the cuff during inflation of the cuff and determine a blood pressure measurement for the subject based on the acquired signal. In some embodiments, the system may comprise a plurality of cuffs as described above. In some embodiments, at least two cuffs of the plurality of cuffs may be of different sizes and the flow resistance of the valve of each of the at least two cuffs may be different.

According to the aspects and embodiments described above, the limitations of existing techniques are addressed. In particular, the above-described aspects and embodiments enable a wider range of flows to be generated to inflate the bladder of the cuff without requiring the hardware configuration of an inflation-based NIBP measurement apparatus to be modified for use with the cuff. In this way, the above-described aspects and embodiments enable inflation-based NIBP measurement for a wider range of cuffs. As a desired fluid (e.g. gas) flow is to a large extent determined by the size of the cuff, the valve disposed along a flow path between the inlet and the bladder or on the bladder according to the above-described aspects and embodiments can be used to optimize the flow range to be supported by a particular size of cuff (e.g. an infant, a child cuff, or any other size cuff). This allows for improved control of the flow range as it can, for example, allow a pump of the inflation-based NIBP measurement apparatus to be operated at a high-flow condition in order to avoid artifacts.

The limitations associated with the existing techniques discussed earlier are therefore addressed by way of the above-described aspects and embodiments.

These and other aspects will be apparent from and elucidated with reference to the embodiment(s) described hereinafter.

## BRIEF DESCRIPTION OF THE DRAWINGS

Exemplary embodiments will now be described, by way of example only, with reference to the following drawings, in which:

> Fig. 1 is a simplified schematic illustration of a cuff according to an embodiment; and
> Fig. 2 is a simplified schematic illustration of a cuff according to another embodiment;
> Fig. 3 is a simplified schematic illustration of a system comprising a cuff according to an embodiment;
> Fig. 4 is a circuit diagram representation of an implementation of a system comprising a cuff according to an embodiment;
> Fig. 5 is a graphical illustration of a flow of fluid through a valve as a function of pressure in a bladder of a cuff;
> Fig. 6(a), (b), (c) and (d) illustrate results of a simulation of flow requirements for an example cuff;
> Fig. 7 is a graphical illustration of a predicted flow of fluid through three different valves as a function of pressure in a bladder of a cuff;
Figs. 8(a), (c) and (e) each illustrate a plurality of inflation-based NIBP measurements for a subject as a function of flow rate using different cuffs; and
Figs. 8(b), (d) and (f) illustrate the corresponding predicted flow of fluid through a valve described herein as a function of pressure in a bladder of the cuff.

DETAILED DESCRIPTION OF EMBODIMENTS

There is provided herein a cuff (or clamp unit) for use with a non-invasive blood pressure (NIBP) measurement apparatus or, more specifically, an inflation-based non-invasive blood pressure (iNIBP) measurement apparatus, which overcomes the limitations with existing techniques.

The cuff described herein can be a wearable cuff. That is, the cuff described herein can be configured to be worn on or around (e.g. wrapped around, attached to, or fastened to) a measurement site of the subject (e.g. a patient). The measurement site of the subject referred to herein can be any site on the body of the subject that is suitable for use in measuring a blood pressure of the subject, such as any site on the body of the subject that comprises an artery. For example, the measurement site of the subject referred to herein may be located on a limb of the subject, such as an arm (e.g. an upper arm or a forearm) of the subject or the leg (e.g. a thigh) of the subject. Thus, the cuff described herein can be configured to be worn on or around (e.g. wrapped around, attached to, or fastened to) a limb of the subject. **The subject referred to herein can be, for example, an adult or a pediatric subject, e.g. an infant, a child or an adolescent.** An infant can, for example, be a neonate, such as a pre-term or premature infant, a full-term infant or a post-term infant.

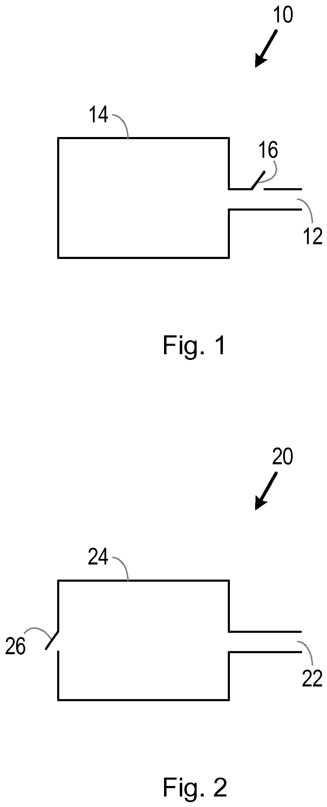

**Figs. 1 and 2** illustrate the cuff 10, 20 described herein. Briefly, with reference to Figs. 1 and 2, the cuff 10, 20 described herein comprises an inlet 12, 22 configured to be coupled to an outlet of the inflation-based NIBP measurement apparatus (not illustrated in Figs. 1 and 2) to receive a flow of fluid.

The cuff 10, 20 described herein also comprises a bladder 14, 24 coupled to the inlet 12, 22 and inflatable to pressurize a measurement site of a subject (not illustrated in Figs. 1 and 2) by receiving the flow of fluid. In this way, the bladder 14, 24 can pressurize an artery in the measurement site of the subject. Typically, the bladder 14, 24 can be supplied with a fluid (e.g. a gas, such as air, or any other fluid) suitable for inflating the bladder 14, 24. The bladder 14, 24 can be inflatable to pressurize the measurement site of a subject (and thus an artery in the measurement site of the subject) at the pressure of the fluid in the bladder 14, 24. Thus, the bladder 34 can hold (or store) at least part of the received fluid.

The cuff 10, 20 described herein also comprises a valve 16, 26. The valve 16, 26 is either disposed along a flow path between the inlet 12 and the bladder 14 (as illustrated in Fig. 1) or disposed on the bladder 24 (as illustrated in Fig. 2). In some embodiments where the valve 16 is disposed along a flow path between the inlet 12 and the bladder 14, such as that illustrated in Fig. 1, the valve 16 may be disposed on a tube (or hose) that is connected to or is part of the bladder 14. The flow resistance of the valve 16, 26 described herein is such that the valve 16, 26 passes (or releases) part of the flow of fluid received in the bladder 14, 24 to the atmosphere in order to inflate the bladder 14, 24 at a required flow rate for inflating the cuff 10, 20. The flow resistance referred to herein can be defined as a pressure drop over the valve 16, 26 divided by a flow rate through the valve 16, 26.

In some embodiments, the flow resistance of the valve 16, 26 can be defined based on a physical property of the cuff 10, 20. For example, in some embodiments, the physical property of the cuff 10, 20 may comprise any one or more of a size of the cuff 10, 20 (e.g. an inner circumference of the cuff 10, 20), an elasticity of the cuff 10, 20, and a compliance of the cuff 10, 20. The compliance of the cuff 10, 20 may also be referred to in the art as the "cuff compliance" for the cuff 10, 20. The cuff compliance for the cuff 10, 20 can be defined as a value that relates a pressure change in the cuff 10, 20 due to a volume change of the cuff 10, 20.

Alternatively or in addition, in some embodiments, the flow resistance of the valve 16, 26 may be defined based on one or more properties of a system (or components of the system) comprising the cuff 10, 20. For example, in some embodiments, the flow resistance of the valve 16, 26 may be defined based on a flow range of a pump configured to output the flow of fluid. For example, the pump configured to output the flow of fluid may have a certain flow range that can be provided. The flow range of the pump referred to herein is the range of flow rates that the pump can provide. In embodiments where a pump is configured to output the flow of fluid, the pump can act as a fluid flow source. As the valve 16, 26 passes part of the flow of fluid received in the bladder 14, 24 to the atmosphere, in effect, the valve 16, 26 passes part of the fluid flow output of the pump to the atmosphere in order to inflate the bladder 14, 24 at a required flow rate for inflating the cuff 10, 20 according to these embodiments.

Alternatively or in addition, in some embodiments, the flow resistance of the valve 16, 26 may be defined based on a target inflation rate for the cuff 10, 20 to reach (or achieve) for determining a blood pressure measurement for the subject or, more specifically, an inflation-based blood pressure measurement.

In some embodiments, the valve 16, 26 may have a diameter that defines the flow resistance of the valve 16, 26. In some embodiments, the valve 16, 26 can be a needle valve, a globe valve, a butterfly valve, a poppet valve or any other valve suitable to pass part of the flow of fluid received in the bladder 14, 24 to the atmosphere.

In some embodiments, the cuff 10, 20 may comprise a deflation valve (not illustrated in Figs. 1 and 2). The deflation valve of the cuff 10, 20 can be controllable to deflate the bladder 14, 24 of the cuff 10, 20. The deflation valve of the cuff 10, 20 can thus be any valve that is controllable to deflate the bladder 14, 24 of the cuff 10, 20. The deflation valve may also be referred to as a release valve.

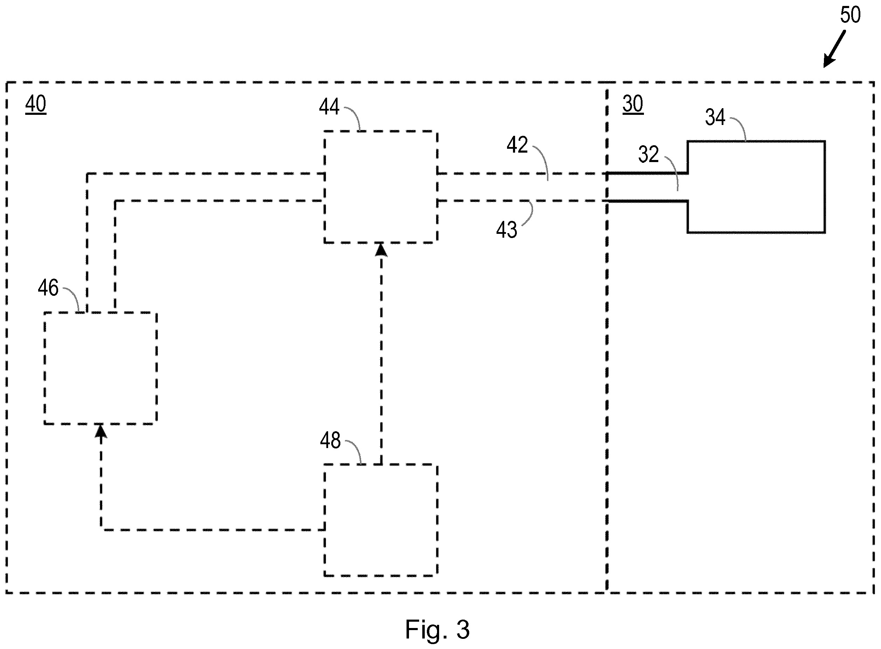

Fig. 3 illustrates a system 50 comprising a cuff 30 according to an embodiment. The system 50 may also be referred to as an inflation-based NIBP measurement system. Although only one cuff 30 is illustrated in Fig. 3, the system 50 can comprise at least one cuff 30, i.e. one or a plurality of cuffs 30. The at least one cuff 30 can comprise at least one cuff 10 as described earlier with reference to Fig. 1. Alternatively or in addition, the at least one cuff 30 can comprise at least one cuff 20 described earlier with reference to Fig. 2.

Thus, the at least one cuff 30 comprises an inlet 32 (such as the inlet 12 illustrated in and described earlier with reference to Fig. 1 or the inlet 22 illustrated in and described earlier with reference to Fig. 2), a bladder 34 (such as the bladder 14 illustrated in and described earlier with reference to Fig. 1 or the bladder 24 illustrated in and described earlier with reference to Fig. 2), and a valve (not illustrated in Fig. 3, such as the valve 16 illustrated in and described earlier with reference to Fig. 1 or the valve 26 illustrated in and described earlier with reference to Fig. 2).

In some embodiments where the system 50 comprises a plurality of cuffs 30, at least two cuffs of the plurality of cuffs 30 may be of different sizes. In these embodiments, the flow resistance of the valve of each of the at least two cuffs can be different.

As illustrated in Fig. 3, in some embodiments, the system 50 can comprise the inflation-based NIBP measurement apparatus 40. The inflation-based NIBP measurement apparatus 40 can be for use with the cuff 30 in measuring blood pressure. As illustrated in Fig. 3, the inflation-based NIBP measurement apparatus 40 comprises an outlet 42. The outlet 42 is configured to be coupled to the inlet 32 of at least one cuff 30 (such as the inlet 12 of the cuff 10 illustrated in Fig. 1 and/or the inlet 22 of the cuff 20 illustrated in Fig. 2).

As illustrated in Fig. 3, in some embodiments, the inflation-based NIBP measurement apparatus 40 may comprise at least one supply line (or at least one supply tube) 43, which may also be referred to as at least one pressure supply line (or at least one pressure supply tube) 43. In these embodiments, the at least one supply line 43 can comprise the outlet 42. Thus, the cuff 30 may be coupled to the inflation-based NIBP measurement apparatus 40 via at least one supply line 43 according to some embodiments. The at least one supply line 43 can be arranged for pressurizing the bladder of the cuff 30 (such as the bladder 14 of the cuff 10 illustrated in Fig. 1 or the bladder 24 of the cuff 20 illustrated in Fig. 2) and, consequently, the measurement site of the subject. The at least one supply line 43 may be provided for inflating and/or deflating the bladder of the cuff 30. As an alternative to the at least one supply line 43, in other embodiments (not illustrated), the inflation-based NIBP measurement apparatus 40 can be coupled directly to (e.g. mounted directly on) the cuff 30. As mentioned earlier, the cuff 30 can be supplied with any fluid suitable for inflating the bladder of the cuff 30.

In some embodiments, as illustrated in Fig. 3, the inflation-based NIBP measurement apparatus 40 may comprise a deflation valve 44. The deflation valve 44 may also be referred to as a release valve. The deflation valve 44 of the inflation-based NIBP measurement apparatus 40 can be controllable to deflate the bladder of the cuff 30 (such as the bladder 14 of the cuff 10 illustrated in Fig. 1 or the bladder 24 of the cuff 20 illustrated in Fig. 2). The deflation valve 44 can thus be any valve that is controllable to deflate the bladder of the cuff 30. The inflation-based NIBP measurement apparatus 40 may comprise the deflation valve 44 instead of or in addition to a deflation valve of at least one the cuff 30. In some embodiments, the deflation valve 44 can be controllable by the inflation-based NIBP measurement apparatus 40 (or, more specifically, by a processor 48 of the inflation-based NIBP measurement apparatus 40 or any other processor) to deflate the bladder of the cuff 30. The inflation-based NIBP measurement apparatus 40 (or the processor 48 of the inflation-based NIBP measurement apparatus 40 or any other processor) may communicate with and/or connect to the deflation valve 44 in any suitable way to control the deflation valve 44.

As illustrated in Fig. 3, in some embodiments, the inflation-based NIBP measurement apparatus 40 may comprise a pump 46. Alternatively or in addition, the pump 46 may be external to (e.g. separate to or remote from) the inflation-based NIBP measurement apparatus 40. The pump 46 can be configured to output a flow of fluid. That is, the pump 46 can be configured to output a flow of fluid via the outlet 42 of the inflation-based NIBP measurement apparatus 40 to the inlet 32 of a cuff 30 to which the outlet 42 is coupled (such as the inlet 12 of the cuff 10 illustrated in Fig. 1 and/or the inlet 22 of the cuff 20 illustrated in Fig. 2).

As described earlier with reference to Figs. 1 and 2, the inlet of the cuff 30 is configured to receive the flow of fluid. Thus, the pump 46 can be controllable to inflate the bladder 34 of the cuff 30 according to some embodiments. The pump 46 can thus be any pump that is controllable to inflate the bladder 34 of the cuff 30. In particular, the pump 46 can be controllable to provide a flow of fluid to inflate the bladder 34 of the cuff 30. In some embodiments, the pump 46 can be controllable by the inflation-based NIBP measurement apparatus 40 (or, more specifically, by a processor 48 of the inflation-based NIBP measurement apparatus 40 or any other processor). The inflation-based NIBP measurement apparatus 40 (or the processor 48 of the inflation-based NIBP measurement apparatus 40 or any other processor) may communicate with and/or connect to the pump 46 in any suitable way to control the pump 46.

In some embodiments, the pump 46 may be configured to provide a flow of fluid at a predetermined margin from a minimum fluid flow limit. By operating the pump 46 at the predetermined margin from the minimum fluid flow limit, noise from the pump 46 that can produce artifacts (which may interfere with inflation-based NIBP measurements) can be prevented from affecting inflation-based NIBP measurements. It can be the case that the artifacts are more of an issue when the pump 46 is operated to provide a flow of fluid at a minimum fluid flow limit of approximately 2 mL/s. Thus, in some embodiments, the pump 46 may be configured to provide a flow of fluid that is greater than 2 mL/s, e.g. greater than 4 mL/s.

The inflation-based NIBP measurement apparatus 40 described herein can be implemented in numerous ways, with software and/or hardware, to perform the various functions described herein. In particular implementations, the inflation-based NIBP measurement apparatus 40 can comprise a plurality of software and/or hardware modules, each configured to perform, or that are for performing, individual or multiple functions described herein.

In some embodiments, the system 50 may comprise a processor 48. As illustrated in Fig. 3, in some embodiments, the inflation-based NIBP measurement apparatus 40 may comprise the processor 48. However, in other embodiments, the processor 48 may be external to (e.g. separate to or remote from) the inflation-based NIBP measurement apparatus 40. The processor 48 may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein.

The inflation-based NIBP measurement apparatus 40 may be implemented as a combination of dedicated hardware (e.g. amplifiers, pre-amplifiers, analog-to-digital convertors (ADCs) and/or digital-to-analog convertors (DACs)) to perform some functions and the processor (e.g. one or more programmed microprocessors, DSPs and associated circuitry) 48 to perform other functions.

In some embodiments, the processor 48 can be configured to acquire a signal indicative of pressure oscillations detected in the cuff 30 during inflation of the cuff 30. In some of these embodiments, the processor 48 can also be configured to determine a blood pressure measurement for the subject based on the acquired signal. Any existing techniques for determining a blood pressure measurement for a subject based on an acquire signal indicative of pressure oscillations can be used and a person skilled in the art will be aware of such existing techniques that may be used.

Although not illustrated in Fig. 3, in some embodiments, the system 50 may comprise at least one pressure sensor. The at least one pressure sensor can be configured to measure the pressure in the bladder of the cuff 30 (such as the bladder 14 of the cuff 10 illustrated in Fig. 1 or the bladder 24 of the cuff 20 illustrated in Fig. 2). In some embodiments, the inflation-based NIBP measurement apparatus 40 may comprise the at least one pressure sensor configured to measure the pressure in the bladder of the cuff 30. Alternatively or in addition, the cuff 30 may comprise the at least one pressure sensor configured to measure the pressure in the bladder of the cuff 30. Alternatively or in addition, at least one pressure sensor external to (e.g. separate to or remote from) the inflation-based NIBP measurement apparatus 40 and/or the cuff 30 may be configured to measure the pressure in the bladder of the cuff 30.

In some embodiments, the at least one pressure sensor can be controllable by the inflation-based NIBP measurement apparatus 40 or, more specifically, the processor 48 of the inflation-based NIBP measurement apparatus 40 or any other processor to measure the pressure in the bladder of the cuff 30. The inflation-based NIBP measurement apparatus 40 (or the processor 48 of the inflation-based NIBP measurement apparatus 40 or any other processor) may communicate with and/or connect to the at least one pressure sensor in any suitable way to control the at least one pressure sensor.

Although also not illustrated in Fig. 3, in some embodiments, the system 50 may comprise a communications interface (or communications circuitry). In some embodiments, the inflation-based NIBP measurement apparatus 40 may comprise a communications interface. Alternatively or in addition, the communications interface may be external to (e.g. separate to or remote from) inflation-based NIBP measurement apparatus 40. The communications interface can be for enabling the inflation-based NIBP measurement apparatus 40, or components of the inflation-based NIBP measurement apparatus 40, to communicate with and/or connect to one or more other components, sensors, interfaces, devices, or memories (such as any of those described herein).

For example, the communications interface can be for enabling the inflation-based NIBP measurement apparatus 40 (or the processor 48 of the inflation-based NIBP measurement apparatus 40 or any other processor) to communicate with and/or connect to any one or more of the deflation valve 44, the pump 46, and the at least one pressure sensor described earlier. The communications interface may enable the inflation-based NIBP measurement apparatus 40, or components of the inflation-based NIBP measurement apparatus 40, to communicate and/or connect in any suitable way. For example, the communications interface may enable the inflation-based NIBP measurement apparatus 40, or components of the inflation-based NIBP measurement apparatus 40, to communicate and/or connect wirelessly, via a wired connection, or via any other communication (or data transfer) mechanism. In some wireless embodiments, for example, the communications interface may enable the inflation-based NIBP measurement apparatus 40, or components of the inflation-based NIBP measurement apparatus 40, to use radio frequency (RF), Bluetooth, or any other wireless communication technology to communicate and/or connect.

Although also not illustrated in Fig. 3, in some embodiments, the system 50 may comprise a memory. In some embodiments, inflation-based NIBP measurement apparatus 40 may comprise the memory. Alternatively or in addition, the memory may be external to (e. g. separate to or remote from) inflation-based NIBP measurement apparatus 40. The inflation-based NIBP measurement apparatus 40 (or the processor 48 of the inflation-based NIBP measurement apparatus 40 or any other processor) may be configured to communicate with and/or connect to the memory. The memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, the memory can be configured to store program code that can be executed by a processor (e.g. the processor 48 of the inflation-based NIBP measurement apparatus 40 or any other processor) to cause the inflation-based NIBP measurement apparatus 40 to operate in the manner described herein.

Alternatively or in addition, in some embodiments, the memory can be configured to store information required by or resulting from implementations of the functions described herein. For example, in some embodiments, the memory may be configured to store any one or more of a measure of a pressure in the bladder of the cuff 30, a determined blood pressure measurement for a subject, or any other information, or any combination of information, required by or resulting from the implementation of the functions described herein. In some embodiments, the inflation-based NIBP measurement apparatus 40 (or the processor 48 of the inflation-based NIBP measurement apparatus 40 or any other processor) can be configured to control the memory to store information required by or resulting from the implementation of the functions described herein.

Although also not illustrated in Fig. 3, the system 50 may comprise a user interface. In some embodiments, the inflation-based NIBP measurement apparatus 40 may comprise the user interface. Alternatively or in addition, the user interface may be external to (e.g. separate to or remote from) the inflation-based NIBP measurement apparatus 40. The inflation-based NIBP measurement apparatus 40 (or the processor 48 of the inflation-based NIBP measurement apparatus 40 or any other processor) may be configured to communicate with and/or connect to a user interface. The user interface can be configured to render (or output, display, or provide) information required by or resulting from the implementation of the functions described herein. For example, in some embodiments, the user interface may be configured to render (or output, display, or provide) one or more of a measure of a pressure in the bladder of the cuff 30, a determined blood pressure measurement for a subject, or any other information, or any combination of information, required by or resulting from the implementation of the functions described herein. Alternatively or in addition, the user interface can be configured to receive a user input. For example, the user interface may allow a user to manually enter information or instructions, interact with and/or control the inflation-based NIBP measurement apparatus 40.

Thus, the user interface may be any user interface that enables the rendering (or outputting, displaying, or providing) of information and, alternatively or in addition, enables a user to provide a user input. For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input. In some embodiments, the inflation-based NIBP measurement apparatus 40 (or the processor 48 of the inflation-based NIBP measurement apparatus 40 or any other processor) can be configured to control the user interface to operate in the manner described herein.

Although also not illustrated in Fig 3, the inflation-based NIBP measurement apparatus 40 may comprise a battery or other power supply for powering the inflation-based NIBP measurement apparatus 40 or means for connecting the inflation-based NIBP measurement apparatus 40 to a mains power supply. It will also be understood that the inflation-based NIBP measurement apparatus 40 may comprise any other component to those described herein or any combination of components.

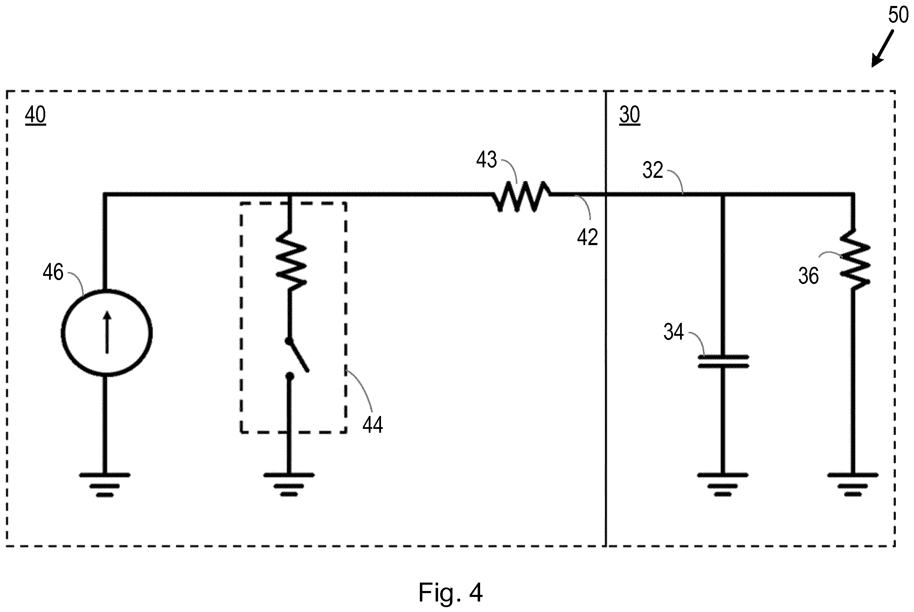

Fig. 4 is a circuit diagram representation of an implementation of a system 50 comprising the cuff 30 described herein according to an example embodiment. The cuff 30 can comprise the cuff 10 described earlier with reference to Fig. 1, the cuff 20 described earlier with reference to Fig. 2, or the cuff 30 described earlier with reference to Fig. 3.

Thus, the cuff 30 illustrated in Fig. 4 comprises an inlet 32 (such as the inlet 12 illustrated in and described earlier with reference to Fig. 1, the inlet 22 illustrated in and described earlier with reference to Fig. 2, or the inlet 32 illustrated in and described earlier with reference to Fig. 3), a bladder 34 (such as the bladder 14 illustrated in and described earlier with reference to Fig. 1, the bladder 24 illustrated in and described earlier with reference to Fig. 2, or the bladder 34 illustrated in and described earlier with reference to Fig. 3), and a valve 36 (such as the valve 16 illustrated in and described earlier with reference to Fig. 1, the valve 26 illustrated in and described earlier with reference to Fig. 2, or the valve 36 illustrated in and described earlier with reference to Fig. 3).

In Fig. 4, the layout of the implementation of system 50 is shown as a circuit diagram comprising connections between a pump 46 of the inflation-based NIBP measurement apparatus 40, a deflation valve 44 of the inflation-based NIBP measurement apparatus 40, a supply line 43 of the inflation-based NIBP measurement apparatus 40, an outlet 42 of the inflation-based NIBP measurement apparatus 40, the inlet 32 of the cuff 30, the bladder 34 of the cuff 30 and the valve 36, which is illustrated as being disposed along a flow path between the inlet 32 of the cuff 30 and the bladder 34 of the cuff 30 in the illustrated example of Fig. 4. The inlet 32 of the cuff 30 is coupled to the outlet 42 of the inflation-based NIBP measurement apparatus 40 to allow the inlet 32 of the cuff 30 to receive a flow of fluid from the pump 46 of the inflation-based NIBP measurement apparatus 40.

In the circuit diagram, the supply line 43 of the inflation-based NIBP measurement apparatus 40, the deflation valve 44 of the inflation-based NIBP measurement apparatus 40 and the valve 36 disposed along a flow path between the inlet 32 of the cuff 30 and a bladder 34 of the cuff 30 are modelled as resistors. More specifically, the supply line 43 of the inflation-based NIBP measurement apparatus 40 is modelled as a series resistance between the outlet 42 of the inflation-based NIBP measurement apparatus 40 and the inlet 32 of the cuff 30. The deflation valve 44 of the inflation-based NIBP measurement apparatus 40 is modelled as a resistor connected to ground (where ground represents the atmosphere) via a switch. This illustrates that the deflation valve 44 of the inflation-based NIBP measurement apparatus 40 can allow a flow of fluid therethrough when the switch is closed. The valve 36 disposed along a flow path between the inlet 32 of the cuff 30 and a bladder 34 of the cuff 30 is modelled as a resistor connected to ground (where ground represents the atmosphere). The valve 36 is modelled as a resistor in parallel to the bladder 34 of the cuff 30. The bladder 34 of the cuff 30 is modelled as a capacitor (as, in effect, the bladder 34 of the cuff 30 stores at least part of the received fluid). The pump 46 is modelled as a current source (as it provides the fluid).

It will be appreciated that the arrangement of components of the system 50 is exemplary and may be different to that show according to other embodiments. For example, the arrangement of the valve 36 and the deflation valve 44 shown in Fig. 4 is exemplary and can be different to that shown according to other embodiments (e.g. any one or more of the valve 36 and the deflation valve 44 may be located nearer to, or be disposed on, the bladder 34 of the cuff 30 according to other embodiments).

The flow of fluid into the bladder 34 of the cuff 30 (via the inlet 32 of the cuff 30) can be defined according to the following equation:

$$q_{provided} = q_{bladder} + q_{valve},$$

where $q_{provided}$ denotes the flow of fluid provided to the inlet 32 of the cuff 30 (e.g. the flow of fluid provided by the pump 46 of the inflation-based NIBP measurement apparatus 40), $q_{bladder}$ denotes the flow of fluid into the bladder 34 of the cuff 30, and $q_{valve}$ denotes the part of the flow of fluid received in the bladder 14, 24 that is passed to the atmosphere by the valve 36.

The flow of fluid $q_{bladder}$ into the bladder 34 of the cuff 30 can be specified by the required inflation rate and the cuff 30 itself. For some cuffs, this flow of fluid $q_{bladder}$ into the bladder 34 of the cuff 30 is too low to be realized by the flow of fluid $q_{provided}$ provided to the inlet 32 of the cuff 30 (e.g. the flow of fluid provided by the pump 46 of the inflation-based NIBP measurement apparatus 40). However, the flow resistance of the valve 36 described herein is such that the valve 36 passes part of the flow of fluid received in the bladder 14, 24 to the atmosphere in order to inflate the bladder 14, 24 at a required flow rate for inflating the cuff 10, 20. Thus, the part of the flow of fluid $q_{valve}$ received in the bladder 14, 24 that is passed to the atmosphere by the valve 36 can ensure that it is possible to provide the total flow of fluid ($q_{bladder} + q_{valve}$) required to inflate the bladder 14, 24 at the required flow rate for inflating the cuff 10, 20 (e.g. by the pump 46 of the inflation-based NIBP measurement apparatus 40).

Thus, as described earlier, the cuff 10, 20, 30 described herein comprises a valve 16, 26, 36 (disposed along a flow path between the inlet 12, 22, 32 and the bladder 14, 24, 34 or disposed on the bladder 14, 24, 34) and the flow resistance of the valve 16, 26, 36 is such that the valve 16, 26, 36 passes part of the flow of fluid received in the bladder 14, 24, 34 to the atmosphere in order to inflate the bladder 14, 24, 34 at a required flow rate for inflating the cuff 10, 20, 30. As the flow resistance of the valve 16, 26, 36 ensures that a sufficient part of the flow of fluid received in the bladder 14, 24, 34 is passed to the atmosphere to enable the bladder 14, 24, 34 to be inflated at a required flow rate for inflating the cuff 10, 20, 30, a valve 16, 26, 36 with optimal flow resistance is provided for the cuff 10, 20, 30. This enables a wide range of fluid flows into the cuff 10, 20, 30 while a pump 46 providing the flow of fluid to the bladder 14, 24, 34 of the cuff 10, 20, 30 can be operated in its usual (e.g. ideal) operating range of fluid flows.

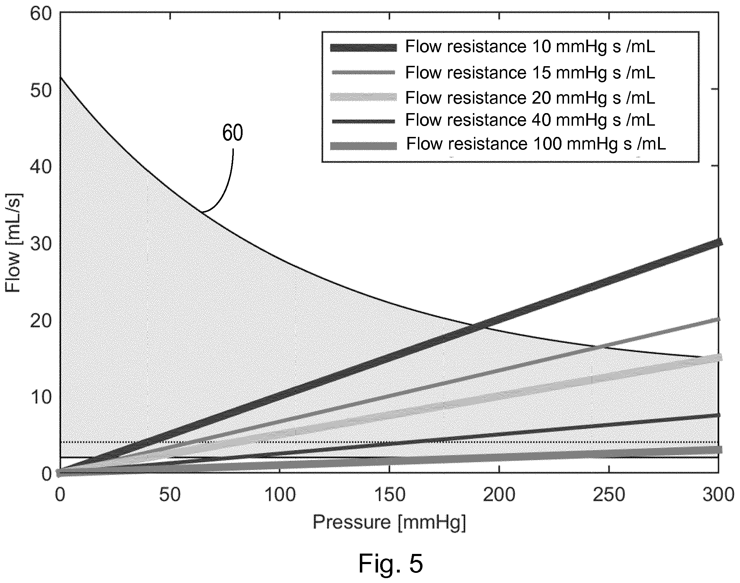

Fig. 5 is a graphical illustration of a flow rate through a valve 16, 26, 36 (measured in mL/s) as a function of pressure in a bladder 14, 24, 34 of a cuff 10, 20, 30 (measured in mmHg). The flow rate through the valve 16, 26, 36 is illustrated on the y-axis and the pressure in the bladder 14, 24, 34 is illustrated on the x-axis. The flow rate through the valve as a function of pressure is illustrated for different flow resistances of the valve 16, 26, 36 (measured in mmHg·s/mL), which are listed in the legend of Fig. 5. The area under the curve 60 is representative of the flow range of the pump 46 that provides the flow of fluid to the bladder 14, 24, 34 of the cuff 10, 20, 30. The flow range of the pump 46 is a range from a minimum flow rate that can be provided by a pump 46 to a minimum flow rate that can be provided by a pump 46. Thus, the curve 60 itself in Fig. 5 represents the maximum flow rate that can be provided by the pump 46.

As illustrated in Fig. 5, if the flow resistance of the valve 16, 26, 36 is too low, the flow rate through the valve 16, 26, 36 to the atmosphere may exceed the maximum flow rate that can be provided by a pump 46 at some pressure level. This can be seen by way of the example illustrated in Fig. 5 where the flow resistance of the valve 16, 26, 36 is 10 mmHg·s/mL. In particular, it can be seen from Fig. 5 that this flow resistance of 10 mmHg·s/mL intersects the maximum flow rate that can be provided by the pump 46 at around 200 mmHg. Consequently, it is not possible to inflate the bladder 14, 24, 34 of the cuff 10, 20, 30 to pressures beyond 200 mmHg, since all of the flow of fluid received in the bladder 14, 24, 34 will be passed to the atmosphere. Thus, it is not possible to further inflate the bladder 14, 24, 34 of the cuff 10, 20, 30 and the pressure in the bladder 14, 24, 34 of the cuff 10, 20, 30 will not increase. On the other hand, if the flow resistance of the valve 16, 26, 36 is too high (e.g. greater than 100 mmHg·s/mL), the flow resistance may not be sufficient to allow the pump to be operated beyond a minimum flow rate that can be provided by a pump 46.

As can be seen in Fig. 5, where the flow resistance of the valve 16, 26, 36 is within the flow range of the pump 46 (around 20 mmHg·s/mL in this illustrated example), the flow rate through the valve 16, 26, 36 to the atmosphere is still close to the maximum flow rate that can be provided by a pump 46 at high pressures. This means that the range of fluid flows that can be provided by the pump 46 is limited. In Fig. 5, this range of fluid flows is represented by the difference between the maximum flow rate that can be provided by the pump 46 and the flow rate through the valve 16, 26, 36. As illustrated in Fig. 5, at 20 mmHg·s/mL and 300 mmHg, this range of fluid flows is limited between 0 and 2 mL/s.

Although this maximum inflation flow restriction is generally not an issue for small cuffs (e.g. for an infant or a smaller subject) as the required inflation flows are also small, it can reduce the inflation speeds that can be achieved in intermediate or larger cuffs (e.g. for a child or adult subject), which in turn can slow down a NIBP measurement and increase the discomfort experienced by the subject.

Therefore, as described herein, the flow resistance of the valve 16, 26, 36 is such that the valve 16, 26, 36 passes part of the flow of fluid received in the bladder 14, 24, 34 to the atmosphere in order to inflate the bladder 14, 24, 34 at a required flow rate for inflating the cuff 10, 20, 30. As the flow resistance of the valve 16, 26, 36 ensures that a sufficient part of the flow of fluid received in the bladder 14, 24, 34 is passed to the atmosphere to enable the bladder 14, 24, 34 to be inflated at a required flow rate for inflating the cuff 10, 20, 30, a valve 16, 26, 36 with optimal flow resistance is provided for the cuff 10, 20, 30. This enables a wide range of fluid flows into the cuff 10, 20, 30 while a pump 46 providing the flow of fluid to the bladder 14, 24, 34 of the cuff 10, 20, 30 can be operated in its usual (e.g. ideal) operating range of fluid flows. In this way, the limitations described earlier can be addressed.

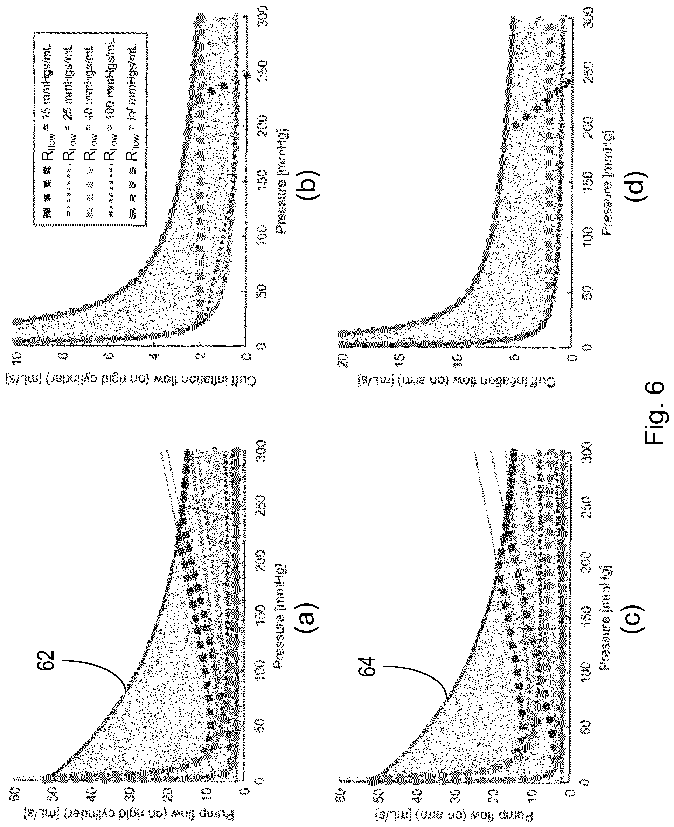

Fig. 6 illustrates results of a simulation of the flow requirements for an example cuff 10, 20, 30. The results of the simulation illustrated in Fig. 6 allow an analysis of the required flow rate for inflating a variety of cuffs 10, 20, 30. Figs. 6(a) and (b) illustrate the case when the cuff is worn on (or, more specifically, wrapped around) a rigid cylinder and Figs. 6(c) and (d) illustrate the case when the cuff is worn on (or, more specifically, wrapped around) an arm of a subject.

In more detail, Figs. 6(a) and (c) illustrate a flow rate provided by a pump 46 (measured in mL/s) as a function of pressure in a bladder 14, 24, 34 of a cuff 10, 20, 30 (measured in mmHg) according to an example. The flow rate provided by the pump ("pump flow") is illustrated on the y-axis and the pressure in the bladder 14, 24, 34 is illustrated on the x-axis in Figs. 6(a) and (c). The area under the curve 62 in Fig. 6(a) and the area under the curve 64 in Fig. 6(c) are representative of the flow range of the pump 46 that provides the flow of fluid to the bladder 14, 24, 34 of the cuff 10, 20, 30. Figs. 6(b) and (d) illustrate a flow rate in the bladder 14, 24, 34 of the cuff 10, 20, 30 (measured in mL/s) as a function of pressure in the bladder 14, 24, 34 of the cuff 10, 20, 30 (measured in mmHg) according to the example. The flow rate in the bladder 14, 24, 34 ("cuff inflation flow") is illustrated on the y-axis and the pressure in the bladder 14, 24, 34 is illustrated on the x-axis in Figs. 6(b) and (d).

The flow rate provided by the pump as a function of the pressure in the bladder 14, 24, 34 in Figs. 6(a) and (c) and the flow rate in the bladder 14, 24, 34 as a function of the pressure in the bladder 14, 24, 34 in Figs. 6(b) and (d) are illustrated for different flow resistances ("$R_{flow}$") of valve 16, 26, 36 (measured in mmHg·s/mL), which are listed in the legend of Fig. 6. The two dashed lines for the flow resistances in Fig. 6 indicate the maximum and minimum flow rates required to inflate the cuff 10, 20, 30.

For infinite resistance (i.e. without a valve 16, 26, 36), the minimum flow rate required to inflate the cuff 10, 20, 30 cannot be provided by the pump 46 for pressures above 30 mmHg. By adding the valve 16, 26, 36 described herein with optimal flow resistance (which in this example is a flow resistance up to 100 mmHg·s/mL), it is possible to achieve the minimum flow rate required to inflate the cuff 10, 20, 30 when the cuff 10, 20, 30 is worn on (or, more specifically, wrapped around) the arm of the subject. With an even lower flow resistance, the minimum flow rate required to inflate the cuff 10, 20, 30 can also be achieved if the cuff 10, 20, 30 is worn on (or, more specifically, wrapped around) the rigid cylinder.

However, if the flow resistance of the valve 16, 26, 36 becomes too low, the desired maximum flow rate required to inflate the cuff 10, 20, 30 can no longer be reached and a maximum inflation pressure limits the system 50. For example, for a valve 16, 26, 36 having a flow resistance of 15 mmHg·s/mL, the cuff 10, 20, 30 cannot be inflated beyond a pressure of 250 mmHg. At this point, all of fluid flow received in the bladder 14, 24, 34 is passed to the atmosphere via the valve 16, 26, 36, such that it is not possible to further inflate the cuff 10, 20, 30. Thus, as described herein, the flow resistance of the valve 16, 26, 36 is such that the valve 16, 26, 36 passes part of the flow of fluid received in the bladder 14, 24, 34 to the atmosphere in order to inflate the bladder 14, 24, 34 at a required flow rate for inflating the cuff 10, 20, 30. In the example described with reference to Fig. 6, this means that the flow resistance of the valve 16, 26, 36 may, for example, be 40 mmHg·s/mL.

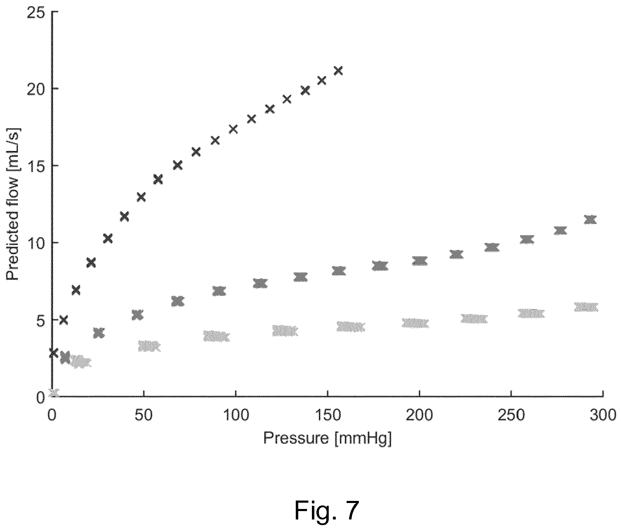

Fig. 7 illustrates a graphical illustration of a predicted flow rate through three different valves 16, 26, 36 (measured in mL/s) as a function of pressure in a bladder 14, 24, 34 of a cuff 10, 20, 30 (measured in mmHg) according to an example. In the example illustrated in Fig. 7, the different valves 16, 26, 36 comprise different needle valves. Each of the valves 16, 26, 36 in this example has a different diameter to define different flow resistances. The flow rate is predicted based on a look-up table previously designed to measure pump flow output as function of pressure, duty cycle, and supply voltage. As illustrated in Fig. 7, it can be seen that different flow resistances of the valves 16, 26, 36 described herein (which are defined by different diameters of valve 16, 26, 36 in this example) result in different flow rates to the atmosphere.

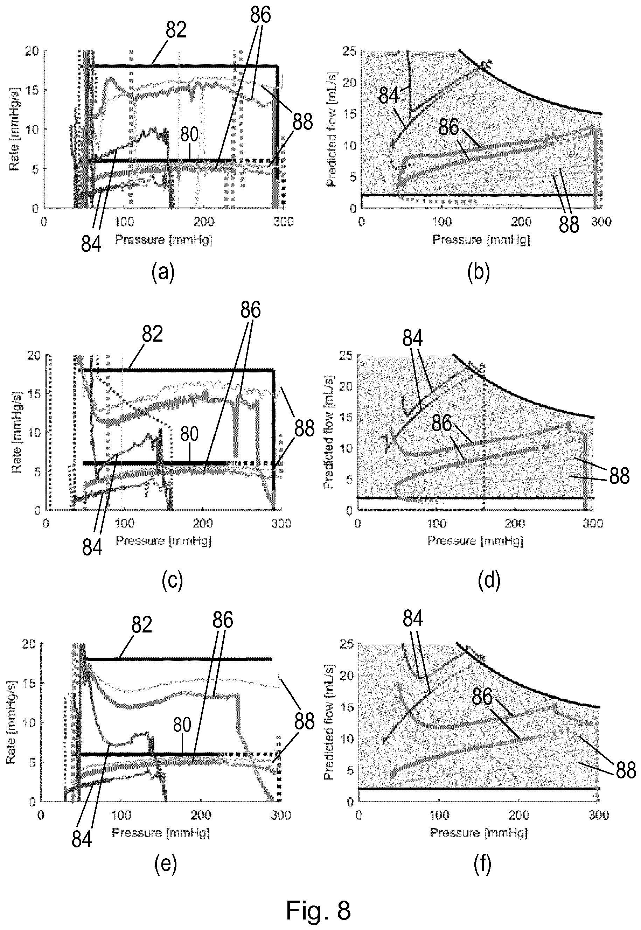

Figs. 8(a), (c) and (e) each illustrate an inflation rate (or pressurization rate, measured in mmHg/s) that can be achieved for a cuff 10, 20, 30 comprising a valve 16, 26, 36 as a function of pressure in a bladder 14, 24, 34 of the cuff 10, 20, 30 (measured in mmHg) according to an example. The pressure is illustrated on the x-axis and the inflation rate ("rate") is illustrated on the y-axis in Figs. 8(a), (c) and (e). Fig. 8(a) illustrates the inflation rate that can be achieved as a function of pressure using an infant cuff 10, 20, 30, Fig. 8(c) illustrates the inflation rate that can be achieved as a function of pressure using a child cuff 10, 20, 30, and Fig. 8(e) illustrates the inflation rate that can be achieved as a function of pressure using a (small) adult cuff 10, 20, 30. The different lines 84, 86, 88 in Figs. 8(a), (c) and (e) represent the inflation rate that can be achieved as a function of pressure using those cuffs 10, 20, 30 comprising different valves 16, 26, 36. Two target inflation rates of 6 mmHg and 18 mmHg are illustrated by the lines 80 and 82 respectively in Figs. 8(a), (c) and (e).

Figs. 8(b), (d) and (f) illustrate the corresponding predicted flow rate through the different valves 16, 26, 36 (measured in mL/s) as a function of pressure in the bladder 14, 24, 34 of the cuffs 10, 20, 30 (measured in mmHg). The predicted flow rate ("predicted flow") is illustrated on the y-axis and the pressure in the bladder 14, 24, 34 is illustrated on the x-axis in Figs. 8(b), (d) and (f). As before, the different valves 16, 26, 36 are represented by the different lines 84, 86, 88 in each of Figs. 8(b), (d) and (f). In the example of Fig. 8, the different cuffs 10, 20, 30 were wrapped around a rigid cylinder to illustrate the results that can be obtained and the different valves 16, 26, 36 comprised different needle valves.

It can be seen from Fig. 8 that the predicted flow rate through the valve 16, 26, 36 illustrated by the line 84 is too large, leading to a maximum pressure in the cuff 10, 20, 30 of about 150 mmHg. The valve 16, 26, 36 illustrated by the line 86 provides adequate results. However, at high pressures, the maximum flow rate is limited and, in some instances, the pressure in a bladder 14, 24, 34 of a cuff 10, 20, 30 does not reach 300 mmHg. This may be acceptable for the infant and child cuffs 10, 20, 30. The valve 16, 26, 36 illustrated by the line 88 provides the optimum results, in particular for the adult cuff 10, 20, 30 comprising the valve 16, 26, 36 illustrated in Figs. 8(e) and (f).

Thus, optimum results can be achieved using this valve 16, 26, 36 disposed along a flow path between the inlet 12, 22, 32 and the bladder 14, 24, 34 of the cuff 10, 20, 30 or disposed on the bladder 14, 24, 34 of the cuff 10, 20, 30 as the flow resistance of the valve 16, 26, 36 is such that the valve 16, 26, 36 passes part of the flow of fluid received in the bladder 14, 24, 34 to the atmosphere in order to inflate the bladder 14, 24, 34 at a required flow rate for inflating the cuff 10, 20, 30 as described herein.

There is thus provided herein a cuff 10, 20, 30 and a system 50 that address the limitations associated with the existing techniques.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A cuff (10, 20, 30) for use with an inflation-based non-invasive blood pressure, NIBP, measurement apparatus (40), the cuff (10, 20, 30) comprising:

   an inlet (12, 22, 32) configured to be coupled to an outlet (42) of the inflation-based NIBP measurement apparatus (40) to receive a flow of fluid; a bladder (14, 24, 34) coupled to the inlet (12, 22, 32) and inflatable to pressurize a measurement site of a subject by receiving the flow of fluid; and a valve (16, 26, 36) disposed along a flow path between the inlet (12, 22, 32) and the bladder (14, 24, 34) or disposed on the bladder (14, 24, 34), wherein the flow resistance of the valve (16, 26, 36) is such that the valve (16, 26, 36) passes part of the flow of fluid received in the bladder (14, 24, 34) to the atmosphere in order to inflate the bladder (14, 24, 34) at a required flow rate for inflating the cuff (10, 20, 30).

2. The cuff (10, 20, 30) as claimed in claim 1, wherein the flow resistance of the valve (16, 26, 36) is defined based on a physical property of the cuff (10, 20, 30).

3. The cuff (10, 20, 30) as claimed in claim 2, wherein the physical property of the cuff (10, 20, 30) comprises any one or more of:

   a size of the cuff (10, 20, 30); an elasticity of the cuff (10, 20, 30); and a compliance of the cuff (10, 20, 30).

4. The cuff (10, 20, 30) as claimed in any of the preceding claims, wherein the flow resistance of the valve is defined based on a flow range of a pump configured to output the flow of fluid.

5. The cuff (10, 20, 30) as claimed in any of the preceding claims, wherein the flow resistance of the valve (16, 26, 36) is defined based on a target inflation rate for the cuff (10, 20, 30) to reach for determining a blood pressure measurement for the subject.

6. The cuff (10, 20, 30) as claimed in any of the preceding claims, wherein the valve (16, 26, 36) has a diameter that defines the flow resistance of the valve (16, 26, 36).

7. The cuff (10, 20, 30) as claimed in any of the preceding claims, wherein the valve (16, 26, 36) is a needle valve, a globe valve, a butterfly valve, or a poppet valve.

8. The cuff (10, 20, 30) as claimed in any of the preceding claims, wherein the cuff (10, 20, 30) comprises:

   a deflation valve controllable to deflate the bladder (14, 24, 34).

9. A system (50) comprising:

   at least one cuff (10, 20, 30) as claimed in any of the preceding claims.

10. The system (50) as claimed in claim 9, wherein the system (50) comprises:

    the inflation-based NIBP measurement apparatus (40).

11. The system (50) as claimed in claim 10, wherein the inflation-based NIBP measurement apparatus (40) comprises:

    a deflation valve (44) controllable to deflate the bladder (14, 24, 34).

12. The system (50) as claimed in claim 10 or 11, wherein the inflation-based NIBP measurement apparatus (40) comprises:

    a pump (46) configured to output the flow of fluid.

13. The system (50) as claimed in any of claims 9 to 12, wherein the system (50) comprises a processor configured to:

    acquire a signal indicative of pressure oscillations detected in the cuff (10, 20, 30) during inflation of the cuff (10, 20, 30); and determine a blood pressure measurement for the subject based on the acquired signal.

14. The system (50) as claimed in any of claims 9 to 13, wherein the system (50) comprises:

    a plurality of cuffs (10, 20, 30) as claimed in any of claims 1 to 8, wherein at least two cuffs of the plurality of cuffs (10, 20, 30) are of different sizes and the flow resistance of the valve of each of the at least two cuffs is different.

10

14

16

12

Fig. 1

20

24

26

22

Fig. 2

Fig. 3

EP 3 628 220 A1

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 18 19 6774

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 450 966 B1 (HANNA D ALAN [US]) 17 September 2002 (2002-09-17) * abstract; figure 1 * * column 5, line 65 - column 7, line 63 * | 1-14 | INV. A61B5/022 A61B5/0225 A61B5/0235 |
| X A | WO 2018/064217 A1 (PHYSIO CONTROL INC [US]) 5 April 2018 (2018-04-05) * abstract; figure 1 * * paragraphs [0022] - [0048] * | 1-9 10-14 | |
| X A | CN 206 315 082 U (SHI ZHEN) 11 July 2017 (2017-07-11) * abstract; claim 1; figure 1 * * paragraph [0014] * | 1,5,8 2-4,6,7, 9-14 | |
| X | DE 22 65 110 A1 (SPEIDEL BLASIUS) 13 January 1977 (1977-01-13) * claim 1; figures 1, 5-8 * | 1-14 | |
| X | US 2012/330112 A1 (LAMEGO MARCELO [US] ET AL) 27 December 2012 (2012-12-27) * abstract; figures 1a-b, 2 * * paragraphs [0008], [0009], [0031] - [0043], [0055] * | 1-14 | TECHNICAL FIELDS SEARCHED (IPC)  A61B |

The present search report has been drawn up for all claims

| Place of search | Date of completion of the search | Examiner |
|---|---|---|
| The Hague | 14 February 2019 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 19 6774

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2019

| Patent document cited in search report | | Publication date | Patent family member(s) | | Publication date |
|---|---|---|---|---|---|
| US 6450966 | B1 | 17-09-2002 | NONE | | |
| WO 2018064217 | A1 | 05-04-2018 | NONE | | |
| CN 206315082 | U | 11-07-2017 | NONE | | |
| DE 2265110 | A1 | 13-01-1977 | NONE | | |
| US 2012330112 | A1 | 27-12-2012 | US | 2012330112 A1 | 27-12-2012 |
| | | | US | 2017196470 A1 | 13-07-2017 |

EPO FORM P0459

For more details about this annex : see Official Journal of the European Patent Office, No. 12/82